# EUROPEAN PATENT APPLICATION

(11) **EP 2 324 860 A2**
(43) Date of publication of application: **25.05.2011**
(21) Application number: 10191869.6
(22) Date of filing: 19.11.2010
(51) Int. Cl.: A61L 15/58

(54) **Medical pressure-sensitive adhesive composition**

(30) Priority: 20.11.2009 JP 2009265718
(71) Applicant: Nitto Denko Corporation, Ibaraki-shi, Osaka 567-8680 (JP)
(72) Inventor: Ishikura, Jun, Osaka 567-8680 (JP); Funayama, Raito, Osaka 567-8680 (JP); Tachikawa, Yu, Osaka 567-8680 (JP); Sekiya, Junichi, Osaka 567-8680 (JP); Kasahara, Tsuyoshi, Osaka 567-8680 (JP); Okamoto, Masayuki, Osaka 567-8680 (JP); Niwa, Masahito, Osaka 567-8680 (JP)
(74) Representative: HOFFMANN EITLE

(57) **Abstract**

There is provided a medical pressure-sensitive adhesive composition including an acrylic copolymer obtained by copolymerizing monomer components containing (a) at least one kind of a monomer of a (meth) acrylic acid alkyl ester and (b) at least one kind of a monomer of an N-hydroxyalkyl (meth) acrylamide, wherein: a content of the (meth)acrylic acid alkyl ester monomer (a) with respect to a total amount of the monomer components is 50 weight% to 99.9 weight and a content of the N-hydroxyalkyl (meth) acrylamide monomer (b) with respect to the total amount is 0.1 weight% to 20 weight%; and the monomer components are substantially free of a monomer having a carboxyl group. The medical pressure-sensitive adhesive composition of the present invention is particularly suitable for applications such as medical patches and patch preparations.

## Description

### BACKGROUND OF THE INVENTION

This application claims priority under 35 U.S.C. Section 119 to Japanese Patent Application No. 2009-265718 filed on November 20, 2009, which is herein incorporated by reference.

### 1. Field of the Invention

The present invention relates to a medical pressure-sensitive adhesive composition, which can be suitably used for a medical patch or patch preparation.

### 2. Description of the Related Art

Patches and patch preparations each of which is used by being attached to a skin for the purposes of, for example, protecting the skin, fixing various medical devices, and transdermally administering a drug are each requested to have the following characteristics. That is, each of the patches and patch preparations shows sufficient pressure-sensitive adhesiveness upon attachment to the skin, and can be released and removed without contaminating the surface of the skin (causing, for example, an adhesive residue or stickiness) after its use. In addition, it is desirable that each of the patches and patch preparations be lowly stimulant to the skin.

Japanese Laid-open Patent Publication No. Hei 4-150865 discloses a patch containing, in its pressure-sensitive adhesive layer, a cross-linked product of a copolymer of: a (meth)acrylic acid alkyl ester or a mixture of the ester and a (meth) acrylic acid alkoxyalkyl ester; and a monomer containing a carboxyl group and/or a hydroxyl group. However, reactivity between an active component such as a drug and a carboxyl group must be taken into consideration because the pressure-sensitive adhesive layer in the above-mentioned patch contains the copolymer having a carboxyl group.

In addition, Japanese Laid-open Patent Publication No. 2003-313122 discloses a patch using an acrylic pressure-sensitive adhesive obtained by polymerizing: a (meth) acrylic acid alkyl ester; and a monomer copolymerizable with the (meth)acrylic acid alkyl ester and free of a carboxyl group and a sulfo group. Further, the pressure-sensitive adhesive layer of the patch in the document contains an organic liquid component. The document describes that the patch shows a small stimulus to a skin while having such a sufficient cohesive strength that no adhesive residue occurs at the time of its release. However, an additional improvement in adhesive property of the pressure-sensitive adhesive free of a carboxyl group and a sulfo group disclosed in the document has been desired because the patch may be released from the skin when the patch is attached to a skin surface for a long time period or attached to a skin surface that moves to a large extent.

### SUMMARY OF THE INVENTION

In view of the foregoing, an object of the present invention is to provide the following medical pressure-sensitive adhesive composition. That is, even when a medical active component such as a drug is incorporated into a pressure-sensitive adhesive layer, the denaturation or the like of the composition due to a reaction with such component can be suppressed. In addition, when the composition is used in a patch or the like, the composition can not only show good fixability for a skin surface by itself but also impart excellent fixability to a medical device such as a catheter, and is hence particularly suitable for applications such as a medical patch.

The inventors of the present invention have made extensive studies for solving the above-mentioned problems. As a result, the inventors have found that the use of an acrylic copolymer obtained by copolymerizing the following monomer components can prevent the denaturation or the like of a pressure-sensitive adhesive composition containing the copolymer due to a reaction with a medical active component and can impart good fixability when the composition is used in a patch or the like. That is, the monomer components contain one or two or more kinds of monomers of (meth) acrylic acid allyl esters and one or two or more kinds of monomers of N-hydroxyalkyl(meth)acrylamides, and are substantially free of a monomer having a carboxyl group. Thus, the inventors have completed the present invention.

A medical pressure-sensitive adhesive composition according to an embodiment of the present invention includes an acrylic copolymer obtained by copolymerizing monomer components containing (a) at least one kind of a monomer of a (meth)acrylic acid alkyl ester and (b) at least one kind of a monomer of an N-hydroxyalkyl(meth)acrylamide, wherein: a content of the (meth)acrylic acid alkyl ester monomer (a) with respect to a total amount of the monomer components is 50 weight% to 99.9 weight% and a content of the N-hydroxyalkyl(meth)acrylamide monomer (b) with respect to the total amount is 0.1 weight% to 20 weight%; and the monomer components are substantially free of a monomer having a carboxyl group.

In a preferred embodiment of the invention, the (meth) acrylic acid alkyl ester (a) has an alkyl group having 1 to 20 carbon atoms.

In a preferred embodiment of the invention, the N-hydroxyalkyl (meth) acrylamide (b) has a hydroxyalkyl group having 2 to 4 carbon atoms.

In a preferred embodiment of the invention, a total content of the (meth)acrylic acid alkyl ester monomer (a) and the N-hydroxyalkyl (moth) acrylamide monomer (b) with respect to the total amount of the monomer components is 60 weight% or more.

In a preferred embodiment of the invention, the (meth) acrylic acid alkyl ester (a) includes at least one selected from the group consisting of butyl acrylate, 2-ethylhexyl acrylate, butyl methacrylate, and 2-ethylhexyl methacrylate.

In a preferred embodiment of the invention, the N-hydroxyalkyl(meth)acrylamide (b) includes at least one selected from the group consisting of N-(2-hyd-roxyethyl)acrylamide and N-(2-hydroxyethyl)methacrylamide.

In a preferred embodiment of the invention, the acrylic copolymer has a glass transition temperature (Tg) of -10°C or less.

When the medical pressure-sensitive adhesive composition of the present invention contains a medical active component such as a drug, the denaturation or the like of the composition due to a reaction with such component is suppressed, and the composition does not threaten to reduce the effectiveness of the above-mentioned medical active component. In addition, when the pressure-sensitive adhesive layer of a patch or patch preparation is formed, the composition can show good fixability for a skin surface and impart excellent fixability to a medical device such as a catheter, and is hence particularly suitable for applications such as a medical patch.

### BRIEF DESCRIPTION OF THE DRAWINGS

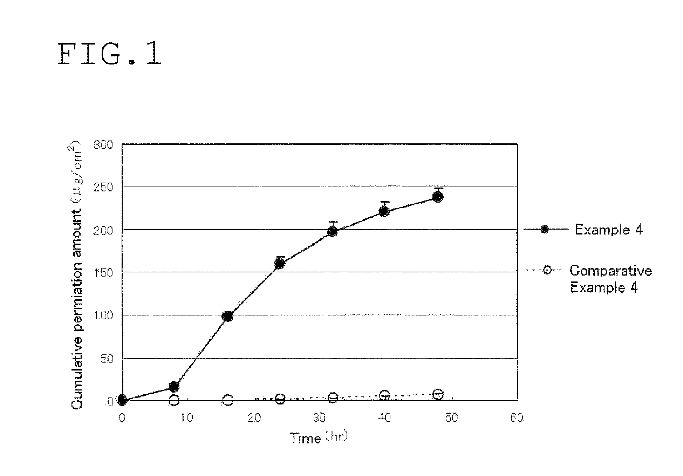

FIG. 1 is a graph illustrating the skin permeability of pramipexole for each of medical pressure-sensitive adhesive compositions of Example 4 of the present invention and Comparative Example 4.

### DETAILED DESCRIPTION OF THE PREFERRED EMBODIMENTS

A medical pressure-sensitive adhesive composition of the present invention includes an acrylic copolymer obtained by copolymerizing monomer components containing (a) one or two or more kinds of monomers of (meth)acrylic acid alkyl esters and (b) one or two or more kinds of monomers of N-hydroxyalkyl (meth) acrylamides, in which the monomer components are substantially free of a monomer having a carboxyl group.

The above-mentioned (meth) acrylic acid alkyl esters (monomers (a)) are typically represented by a formula (I).

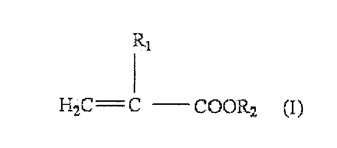

In the formula, R₁ represents a hydrogen atom or a methyl group, and R₂ represents an alkyl group. The alkyl group is preferably an alkyl group having 1 to 20 carbon atoms. The alkyl group may be linear, branched, or cyclic.

Examples of the above-mentioned (meth) acrylic acid alkyl ester include methyl (meth)acrylate, ethyl (meth) acrylate, n-propyl (meth)acrylate, isobutyl (meth)acrylate, sec-butyl (meth)acrylate, t-butyl (meth)acrylate, pentyl (meth)acrylate, isopentyl (meth)acrylate, hexyl (meth)acrylate, heptyl (meth)acrylate, n-octyl (meth) acrylate, isooctyl (meth)acrylate, 2-ethylhexyl (meth)acrylate, nonyl (meth)acrylate, isononyl (meth)acrylate, decyl (meth)acrylate, isodecyl (meth)acrylate, undecyl (meth)acrylate, dodecyl (meth)acrylate, tridecyl (meth)acrylate, tetradecyl (meth)acrylate, pentadecyl (meth)acrylate, hexadecyl (meth)acrylate, heptadecyl (meth)acrylate, octadecyl (meth) acrylate, nonadecyl (meth) acrylate, eicosyl (meth) acrylate, cyclopentyl (meth)acrylate, cyclohexyl (meth)acrylate, bornyl (meth)acrylate, and isobornyl (meth)acrylate. They may be used alone or in combination.

Of the above-mentioned esters, a monomer component that reduces a glass transition temperature is preferably used in order that pressure-sensitive adhesiveness may be imparted at normal temperature. A (meth)acrylic acid alkyl ester in which the alkyl group represented by R₂ in the formula (I) has 2 to 14 carbon atoms is more preferred, and a (meth)acrylic acid alkyl ester in which the alkyl group has 2 to 10 carbon atoms is still more preferred. To be specific, butyl acrylate, 2-ethylhexyl acrylate, butyl methacrylate, 2-ethylhexyl methacrylate, or the like is preferred, and 2-ethylhexyl acrylate is most preferred. This is because of the following reasons. That is, a polymer having a sufficiently low glass transition temperature (-70°C) is obtained when the ester is polymerized. In addition, the ester is easily available. It should be noted that, in the present invention, the above-mentioned ester having an alkyl group having 2 to 10 carbon atoms accounts for preferably about 70 weight or more, more preferably about 90 weight% or more, of the total amount of the monomers (a).

In the present invention, the content of the monomers (a) is 50 weight% to 99.9 weight%, preferably about 60 weight% to about 99 weight%, more preferably about 70 weight% to about 99 weight%, still more preferably about 85 weight% to about 97 weight% with respect to the total amount of the monomer components. When the content of the monomers (a) falls within such range, the pressure-sensitive adhesive performance (such as adhesion or tack) of a pressure-sensitive adhesive layer formed of the pressure-sensiti ve adhesive composition becomes good. On the other hand, when the content of the monomers (a) exceeds 99.9 weight%, the amount of the monomers (b) that can be incorporated into the monomer components reduces, and hence it may become difficult to achieve compatibility between a cohesive strength (such as durability against release in the presence of a certain stress, that is, constant-load property) and repulsion resistance. It should be noted that the composition of the above-mentioned monomer components (monomer composition) typically corresponds to the composition of the respective constituent monomers of the acrylic copolymer obtained by polymerizing the monomer components in general.

The N-hydroxyalkyl(meth)acrylamides (monomers (b)) are typically represented by the following formula (II).

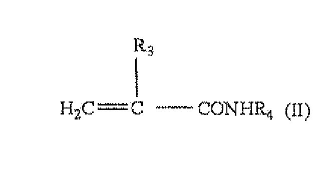

In the formula, R₃ represents a hydrogen atom or a methyl group, and R₄ represents a hydroxyalkyl group.

As the above-mentioned hydroxyalkyl group in the formula (II) , a hydroxyalkyl group having 2 to 4 carbon atoms is preferred. The alkyl group in the above-mentioned hydroxyalkyl group may be linear or branched. Examples of the N-hydroxyalkyl(meth)acrylamide represented by the formula (II) include N-(2-hydroxyethyl)acrylamide, N-(2-hydroxyethyl)methacrylamide, N-(2-hydroxypropyl)acrylamide, N-(2-hydroxypropyl)methacrylamide, N-(1-hydroxypropyl)acrylamide, N-(1-hydroxypropyl)methacrylamide, N-(3-hydroxypropyl)acrylamide, N-(3-hydroxypropyl)methacrylamide, N-(2-hydroxybutyl)acrylamide, N-(2-hydroxybutyl)methacrylamide, N-(3-hydroxybutyl)acrylamide, N-(3-hydroxybutyl)methacrylamide, N-(4-hydroxybutyl)acrylamide, and N-(4-hydroxybutyl)methacrylamide. They may be used alone or in combination. Preferred examples of the monomers (b) in the present invention include N-(2-hydroxyethyl)acrylamide and N-(2-hydroxyethyl)methacrylamide. A particularly preferred example of the monomers (b) is N- (2-hydroxyethyl) acrylamide (HERAA). This is because it is possible to form a pressure-sensitive adhesive layer having good hydrophilic and hydrophobic balance and having excellent pressure-sensitive adhesiveness balance. For example, HEAA accounts for preferably 50 weight% or more, more preferably 70 weight% or more, still more preferably substantially all of the monomers (b).

The monomers (b) can contribute to an improvement in cohesiveness of a pressure-sensitive adhesive by virtue of an interaction between their molecules. In the present invention, the content of the monomers (b) is 0.1 weight% to 20 weight%, preferably 1 weight% to 15 weight% with respect to the total amount of the monomer components. As long as the content of the monomers (b) falls within such range, a sufficient cohesive strength can be imparted to the pressure-sensitive adhesive, and hence the following risks can be avoided. That is, an adhesive lies off an end face of a patch toward the outside owing to the movement of a skin to cause the contamination of a garment or the like, or an adhesive residue occurs on the skin at the time of the release of the patch. On the other hand, when the content of the monomers (b) exceeds 20 weight% , tack may reduce to cause, for example, the floating, or the release of an end, of the patch in the case of an adherend having a rough surface and stretching property such as a skin.

In a preferred embodiment of the pressure-sensitive adhesive composition of the present invention, the content of the monomers (b) is preferably about 2 weight% or more (typically 2 weight% to 20 weight%), more preferably about 3 weight% or more (typically 3 weight% to 15 weight%, e.g. , 3 weight% to 12 weight%) with respect to the total amount of the monomer components. With a pressure-sensitive adhesive composition containing an acrylic copolymer obtained by copolymerizing the monomer components containing the monomers (b) at such ratio, even in, for example, the case where the monomer components are substantially free of a monomer having a heteroatom other than oxygen (such as nitrogen or sulfur) except the monomers (b) , a cohesive strength and an adhesive strength when the composition is bonded to a skin surface can be additionally improved. The phrase "substantially free of a monomer having a heteroatom" as used in the specification comprehends not only the case where the content of the monomer having a heteroatom is zero but also the case where the content is 0.1 weight% or less with respect to the total amount of the monomer components.

A mass ratio (a:b) between the monomers (a) and (b) in the monomer components is, for example, 99.9:0.1 to 71:29, preferably 99:1 to 75:25, more preferably 98:2 to 80:20, still more preferably 97:3 to 85:15. As long as the mass ratio (a:b) falls within such range, a patch or the like having an additionally good cohesive strength and an additionally good adhesive strength when bonded to a skin surface can be obtained even in, for example, the case where the monomer components are of such composition as to be substantially free of a monomer having a heteroatom other than oxygen (such as nitrogen or sulfur) except the monomers (b) (e.g., the monomer components are substantially composed of the monomers (a) and (b)).

The total content of the monomers (a) and (b) is preferably about 60 weight% or more, more preferably about 80 weight% or more, still more preferably about 90 weight% or more, particularly preferably about 95 weight% or more with respect to the total amount of the monomer components. In a preferred embodiment of the pressure-sensitive adhesive composition of the present invention, the monomer components are substantially composed of the monomers (a) and (b) (that is, the total content of the monomers (a) and (b) substantially accounts for 100 weight% of all the monomer components). With such pressure-sensitive adhesive composition, a patch or the like having a good cohesive strength and a good adhesive strength when bonded to a skin surface can be obtained.

In the present invention, the above-mentioned monomer components are characterized by being substantially free of a monomer having a carboxyl group. The term "monomer having a carboxyl group" as used herein typically refers to, for example, an ethylenically unsaturatedmonomer having at least one carboxyl group in its molecule (the carboxyl group may be in a form of an anhydride) (typically a vinyl-based monomer). Examples of such monomer having a carboxyl group include: ethylenically unsaturated monocarboxylic acids such as (meth) acrylic acid and crotonic acid; ethylenically unsaturated dicarboxylic acids such as maleic acid, itaconic acid, and cit raconic acid; and anhydrides of ethylenically unsaturated dicarboxylic acids such as maleic anhydride and itaconic anhydride. It should be noted that the phrase "the monomer components are substantially free of a monomer having a carboxyl group" as used in the specification comprehends not only the case where the monomer components subjected to copolymerization are completely free of the monomer having a carboxyl group but also the case where the content of the monomer is 0.1 weight% or less with respect to the total amount of the monomer components.

Further, in the present invention, it is preferred that the above-mentioned monomer components not only be substantially free of a monomer having a carboxyl group but also be substantially free of a monomer having an acidic group other than a carboxyl group (such as a sulfo group or a phosphate group). That is, it is preferred that the above-mentioned monomer components be completely free of the monomer having a carboxyl group and the monomer having any other acidic group or contain these monomers at a content of 0.1 1 weight% or less with respect to their total amount. The use of such monomer components can forestall, for example, a reaction between the above-mentioned carboxyl group or the like and a medical active component such as a drug when the above-mentioned active component is incorporated into a pressure-sensitive adhesive layer formed by using the pressure-sensitive adhesive composition.

Any other monomer (c) as an optional component as well as the monomers (a) and (b) can be incorporated into the above-mentioned monomer components. The use of such monomer (c) enables additionally proper control of, for example, various properties of a pressure-sensitive adhesive and the structure of the acrylic copolymer. Any appropriate monomer copolymerizable with the above-mentioned monomers (a) and (b), and free of a carboxyl group, preferably a carboxyl group and any other acidic group can be adopted as the monomer (c). For example, monomers each having one or two or more ethylenically unsaturated groups such as a (meth)acryloyl group and a vinyl group can each be used. Such monomers may be used alone or in combination.

Further, as the monomer (c), monomers each containing nitrogen (N) as a constituent atom (however, not including those compounds corresponding to the monomers (b)) may be used. Examples of such monomers each containing a nitrogen atom include cyclic (meth)acrylamides such as N-(meth)acryloylmorpholine and N-acryloylpyrrolidine: (meth)acrylamide; non-cyclic (meth)acrylamides such as N-substituted (meth)acrylamides (including: N-alkyl(meth)acrylamides such as N-ethyl(meth)acrylamide and N-n-butyl (meth) acrylamide; N,N-dialkyl (meth) acrylamides such as N,N-dimethyl (meth) acrylamide, N, N-diethyl (meth) acrylamide, N,N-dipropyl (meth) acrylamide, N,N-diisopropyl(meth)acrylamide, N,N-di(n-butyl) (meth)acrylamide, N,N-di(t-butyl) (meth)acrylamide); N-vinyl cyclic amides such as N-vinyl-2-pyrrolidone, N-vinyl-2-piperidone, N-vinyl-3-morpholinone, N-vinyl-2-caprolactam, N-vinyl-1,3-oxazin-2-one, N-vinyl-3,5-morpholinedione; monomers each having an amino group such as aminoethyl (meth)acrylate, N,N-dimethylaminoethyl (meth) acrylate, and N,N-dimethylaminopropyl (meth)acrylate; monomers each having a maleimide skeleton such as N-cyclohexylmaleimide and N-phenylmaleimide; and itaconimide-based monomers such as N-methylitaconimide, N-ethylitaconimide, N-butylitaconimide, N-2-ethylhexylitaconimide, N-laurylitaconimide, and N-cyclohexylitaconimide. They may be used alone or in combination.

Other examples of monomers which can be adopted as the monomer (c) include monomers each having an epoxy group such as glycidyl (meth)acrylate and allyl glycidyl ether; monomers each having an alkoxy group such as methoxyethyl (meth)acrylate, methoxypropyl (meth) acrylate, methoxyethylene glycol (meth)acrylate, and methoxypolypropylene glycol (meth) acrylate; monomers each having a cyano group such as acrylonitrile and methacrylonitrile; styrene-based monomers such as styrene and α-methylstyrene; α-olefins such as ethylene, propylene, isoprene, butadiene, and isobutylene; monomers each having an isocyanate group such as 2-methacryloyloxyethyl isocyanate; vinylester-based monomers such as vinyl acetate and vinyl propionate; vinyl ether-based monomers such as vinyl ether; (meth) acrylates each having a heterocycle such as tetrahydrofurfuryl (meth) acrylate; monomers each having a halogen atom such as a fluorinated (meth) acrylate; monomers each having an alkoxysilyl group such as 3-methacryloxypropyltrimethoxysilane and vinyltrimethoxysilane; monomers each having a siloxane bond such as silicone (meth)acrylate; and (meth)acrylates each having an aromatic hydrocarbon group such as phenyl (meth) acrylate, benzyl (meth) acrylate, phenoxyethyl (meth)acrylate, and phenoxydiethylene glycol (meth) acrylate.

Further, as the monomer (c), there may be used, for example, polyfunctional monomers such as ethylene glycol di (meth) acrylate, diethylene glycol di(meth)acrylate, triethylene glycol di(meth) acrylate, tetraethylene glycol di(meth)acrylate, polyethylene glycol di(meth) acrylate, polypropylene glycol di(meth) acrylate, neopentyl glycol di (meth) acrylate, hexanediol di(meth)acrylate, pentaerythritol di(meth)acrylate, trimethylolpropane tri(meth) acrylate, pentaerythritol tri(meth) acrylate, dipentaerythritol hexa(meth) acrylate, epoxy acrylate, polyester acrylate, urethane acrylate, divinylbenzene, butyl di(meth) acrylate, and hexyl di(meth)acrylate.

Still other examples of the monomer (c) include monomers each having a hydroxyl group such as: hydroxyalkyl (meth) acrylates including 2-hydroxyethyl (meth)acrylate, 2-hydroxypropyl (meth) acrylate, 3-hydroxypropyl (meth)acrylate, 2-hydroxybutyl (meth)acrylate, 4-hydroxybutyl (meth)acrylate, 6-hydroxyhexyl (meth) acrylate, 8-hydroxyoctyl (meth)acrylate, 10-hydroxydecyl (meth) acrylate, 12-hydroxylauryl (meth)acrylate, and [4 - (hydroxymethyl) cyclohexyl] (meth) acrylate; and alkenyl alcohols such as vinyl alcohol and allyl alcohol.

It should be noted that, when a monomer having a hydroxyl group is used as the monomer (c), such monomer (c) having a hydroxyl group is preferably used at a content smaller than that of the monomers (b) lest an effect obtained by using the monomers (b) should be impaired. In other words, the content of the monomers (b) is preferably more than 50 weight%, more preferably 60 weight% or more, still more preferably 75 weight% or more, particularly preferably 90 weight% or more with respect to the total amount of the monomers each having a hydroxyl group in the monomer components. Alternatively, the monomers each having a hydroxyl group in the monomer components may be substantially only the monomers (b).

Of the above-mentioned monomers, a vinyl-based monomer is preferably used as the monomer (c), and a vinyl-based monomer having a heterocycle containing a nitrogen atom is more preferably used as the monomer.

The content of the monomer (c) (when two or more kinds of monomers are incorporated, the total content of the monomers) is preferably 0 weight% to 40 weight%, more preferably 1 weight% to 35 weight%, and still more preferably 5 weight% to 30 weight% with respect to the total amount of the monomer components. When the content of the monomer (c) is excessively large, pressure-sensitive adhesive performance in a pressure-sensitive adhesive layer formed by using the resultant pressure-sensitive adhesive composition may be apt to lose its balance.

The above-mentioned monomer components can contain the above-mentioned monomers so that the resultant acrylic copolymer may have a glass transition temperature (Tg) of preferably -10°C or less (typically -10°C to -70°C) and more preferably -20°C or less (typically -20°C to -70°C). In other words, the composition of the monomer components can be adjusted so that the Tg may fall within the above-mentioned range. Here, the Tg of the acrylic copolymer is a value determined from Fox's equation on the basis of the Tg's of homopolymers of the respective monomers constituting the monomer components and the mass fraction of each monomer (copolymer composition). Values for the Tg's of the homopolymers of the respective monomers can be obtained from various known documents (such as "HANDBOOK OF PRESSURE-SENSITIVE ADHESIVE TECHNOLOGY published by THE NIKKAN KOGYO SHIMBUN, LTD.).

In the present invention, a polymerization method for obtaining the acrylic copolymer from the above-mentioned monomer components is not particularly limited, and any appropriate polymerization method can be adopted. For example, a polymerization method involving the use of a thermal polymerization initiator (a thermal polymerization method such as a solution polymerization method, an emulsion polymerization method, or a bulk polymerization method), or a polymerization method involving applying an active energy ray (also referred to as "high-energy ray") such as light or radiation can be adopted.

Of the above-mentioned polymerization methods, the solution polymerization method can be preferably adopted because the method is excellent in, for example, workability and quality stability. The mode of the solution polymerization is not particularly limited, and any appropriate mode can be adopted. To be specific, any appropriate monomer supply method, polymerization conditions (such as a polymerization temperature, a polymerization time, and a polymerization pressure), and materials to be used (such as a polymerization initiator and a surfactant) can be adopted. Any one of, for example, a batch loading system involving supplying the total amount of the monomer components to a reaction vessel in one stroke, a continuous supply (dropping) system, and a split supply (dropping) system can be adopted as the above-mentioned monomer supply method. A preferred mode is, for example, such a mode that a solution prepared by dissolving the total amount of the monomer components and an initiator in a solvent is supplied to the reaction vessel and then the monomer components are collectively polymerized (batch polymerization). Such batch polymerization is preferred because a polymerization operation and process control are easy. Another preferred mode is, for example, such a mode that an initiator (typically a solution prepared by dissolving the initiator in a solvent) is prepared in a reaction vessel and then a solution prepared by dissolving the monomer components in a solvent is polymerized while being dropped into the reaction vessel (dropping polymerization or continuous polymerization). Part of the monomer components (part of the components and/or part of the amount) may be loaded into the reaction vessel typically together with a solvent, and the remaining monomer components may be dropped into the reaction vessel. When the monomer components containing the monomers (b) at a content of 15 weight% or more are polymerized, the dropping polymerization is more preferably employed from the viewpoint of the ease with which a polymerization reaction is uniformly advanced.

Examples of the above-mentioned thermal polymerization initiator include: azo-based compounds (azo-based initiators) such as 2,2'-azobisisobutyronitrile, 2,2'-azobis-2-methylbutyronitrile, dimethyl 2,2'-azcbis (2-methyl-propionate), 4,4'-azobis-4-cyanovaleric acid, azobisisovaleronitrile, 2,2'-azobis(2-amidinopropane) dihydrochloride, 2,2'-azobis[2-(5-methyl-2-imidazolin-2-yl)propane] dihydrochloride, 2,2'-azobis(2-methylpropionamidine) disulfate, 2,2'-azobis(N,N'-dimethyleneisobutylamidine) dihydrochloride, and 2,2'-azobis[N-(2-carboxyethyl)-2-methylpropionamidine] hydrate; persulfates such as potassium persulfate and ammonium persulfate; peroxides (peroxide-based initiators) such as dibenzoyl peroxide, tert-butyl permaleate, t-butyl hydroperoxide, and hydrogen peroxide; substituted ethane-based initiators such as phenyl-substituted ethane; and redox-type initiators such as a mixed agent of a persulfate and sodium hydrogen sulfite, and a mixed agent of a peroxide and sodium ascorbate. When monomer components are polymerized by a thermopolymerization method, the polymerization temperature is preferably about 20 °C to about 100 °C, more preferably about 40°C to about 80°C.

A polymerization method involving applying light (typically UV light) is typically performed by using a photopolymerization initiator. The photopolymerization initiator is not particularly limited, and for example, a ketal-based photopolymerization initiator, an acetophenone-based photopolymerization initiator, a benzoin ether-based photopolymerization initiator, an acylphosphine oxide-based photopolymerization initiator, an α-ketol-based photopolymerization initiator, an aromatic sulfonyl chloride-based photopolymerization initiator, an optically active oxime-based photopolymerization initiator, a benzoin-based photopolymerization initiator, a benzyl-based photopolymerization initiator, a benzophenone-based photopolymerization initiator, or a thioxanthone-based photopolymerization initiator can be used. Such photopolymerization initiators may be used alone or in combination.

Examples of the ketal-based photopolymerization initiator include 2,2-dimethoxy-1,2-diphenylethan-1-one [such as one under the trade name "Irgacure 651" (manufactured by Ciba Japan KK)]. Examples of the acetophenone-based photopolymerization initiator include 1-hydroxycyclohexyl phenyl ketone [such as one under the trade name "Irgacure 184" (manufactured by Ciba Japan KK)], 2,2-diethoxyacetophenone, 2,2-dimethoxy-2-phenylacetophenone, 4-phenoxydichloroacetophenone, and 4-(t-butyl) dichloroacetophenone. Examples of the benzoin ether-based photopolymerization initiator include benzoin methyl ether, benzoin ethyl ether, benzoin propyl ether, benzoin isopropyl ether, and benzoin isobutyl ether. Examples of the acylphosphine oxyde-based photopolymerization initiator include one under the trade name "Lucirin TPO" (manufactured by BASF). Examples of the α-ketol-based photopolymerization initiator include 2-methyl-2-hydroxypropiophenone and 1-[4-(2-hydroxyethyl)phenyl]-2- methylpropan-1-one. Examples of the aromatic sulfonyl chloride-based photopolymerization initiator include 2-naphthalenesulfonyl chloride. Examples of the optically active oxime-based photopolymerization initiator include 1-phenyl-1,1-propanedione-2-(o-ethoxycarbonyl)-oxime. Examples of the benzoin-based photopolymerization initiator include benzoin. Examples of the benzyl-based photopolymerization initiator include benzyl. Examples of the benzophenone-based photopolymerization initiator include benzophenone, benzoylbenzoic acid, 3,3'-dimethyl-4-methoxybenzophenone, polyvinylbenzophenone, and a-hydroxycyclohexyl phenyl ketone. Examples of the thioxanthone-based photopolymerization initiator include thioxanthone, 2-chlorothioxanthone, 2-methylthioxanthone, 2,4-dimethylthioxanthone, isopropylthioxanthone, 2,4-diisopropylthioxanthone, and dodecylthioxanthone.

The usage of the above-mentioned polymerization initiator is not particularly limited. For example, the usage of the polymerization initiator is preferably about 0.01 part by weight to about 2 parts by weight, more preferably about 0. 01 part by weight to about 1 part by weight with respect to 100 parts by weight of the total amount of the monomer components .

The medical pressure-sensitive adhesive composition of the present invention can further contain any appropriate tackifier in addition to the above-mentioned acrylic copolymer. Examples of such tackifier include: natural products such as a rosin-based resin and a terpene-based resin, and derivatives of the natural products; and synthetic resins such as an aliphatic petroleum resins, an alicyclicpetroleumresin, e.g., an alicyclic, saturated hydrocarbon resin, a petroleum resin, an aromatic petroleum resin, a coumarone-indene resin, a styrene-based resin, a phenol-based resin, and a xylene-based resin. Any such tackifier can be incorporated at a content in the range of 1 weight% to 30 weight% with respect to the total amount of the pressure-sensitive adhesive composition.

In addition, the medical pressure-sensitive adhesive composition of the present invention can be appropriately compounded with a polymer other than the above-mentioned acrylic copolymer for a viscosity adjustment (typically thickening) purpose. As such polymer for viscosity adjustment, there may be used, for example, styrene butadiene rubber (SBR), isoprene rubber (1R), a styrene-butadiene-styrene block copolymer (SBS), an ethylene-vinyl acetate copolymer, acrylic rubber, polyurethane, or polyester. Further, an acrylic copolymer obtained by copolymerizing a (meth) acrylic acid alkyl ester with a functional monomer (for example, one or two or more kinds selected from acrylic monomers each having a functional group such as acrylamide, acrylonitrile, acryloylmorpholine, and acrylic acid) may be used. It should be noted that a polymer for viscosity adjustment substantially free of a carboxyl group, more preferably a carboxyl group and any other acidic group is preferably adopted lest the characteristics of the present invention should be impaired. Those polymers for viscosity adjustment may be used alone or in combination.

The content of the above-mentioned polymer for viscosity adjustment in the medical pressure-sensitive adhesive composition of the present invention is preferably about 40 weight% or less, more preferably about 1 weight% to about 40 weight%, more preferably about 5 weight% to about 20 weight%.

The medical pressure-sensitive adhesive composition of the present invention can further contain an organic liquid component having compatibility with the above-mentioned acrylic copolymer. The organic liquid component can plasticize the pressure-sensitive adhesive composition to provide a feeling of softness. As a result, when the pressure-sensitive adhesive composition of the present invention is used as a pressure-sensitive adhesive layer, pain or skin irritation resulting from a skin adhesive strength can be reduced upon release of a patch or patch preparation such as a pressure-sensitive adhesive tape or a transdermally absorbable preparation from a skin. Therefore, any component can be used as the organic liquid component without any particular limitation as long as the component has a plasticizing action. It should be noted that a component having an absorption-promoting action is preferably used for improving transdermal absorption property when a drug is incorporated into the pressure-sensitive adhesive layer.

Examples of the above-mentioned organic liquid component include: plant fats and oils such as olive oil, castor oil, and palm oil; animal fats and oils such as lanolin; organic solvents such as dimethyl decyl sulfoxide, methyl octyl sulfoxide, dimethyl sulfoxide, dimethylformamide, dimethylacetamide, dimethyllaurylamide, methylpyrrolidone, and dodecylpyrrolidone; liquid surfactants such as a polyoxyethylene sorbitan fatty acid ester, a sorbitan fatty acid ester, and a polyoxyethylene fatty acid ester; plasticizers such as diisopropyl adipate, a phthalate, and diethyl sebacate; hydrocarbons such as squalane and liquid paraffin; fatty acid alkyl esters such as ethyl oleate, isopropyl palmitate, octyl palmitate, isopropyl myristate, isotridecyl myristate, and ethyl laurate; fatty acid esters of polyhydric alcohols such as a glycerin fatty acid ester and a propylene glycol fatty acid ester; ethoxylated stearyl alcohol; and a pyrrolidone carboxylic acid fatty acid ester. They may be used alone or in combination.

In addition, the medical pressure-sensitive adhesive composition of the present invention may further contain any other components as far as the features of the present invention are not impaired. Examples of such components include antioxidants such as ascorbic acid, tocopherol acetate, natural vitamin E, dibutylhydroxytoluene, and butylhydroxyanisole; arnine-ketone-based anti-aging agents such as 2,6-tert-butyl-4-methylphenol; aromatic secondary amino-based anti-aging agents such as N,N'-di-2-naphthyl-p-phenylenediamine; monophenol-based anti-aging agents such as a 2,2,4-trimethyl-1,2-dihydroquinoline polymer; bisphenol-based anti-aging agents such as 2,2'-methylenebis(4-ethyl-6-tert-butylphenol); polyphenol-based anti-aging agents such as 2, 5-tert-butylhydroquinone; fillers such as kaolin, hydrous silicon dioxide, zinc oxide, and starch acrylate 1000; softening agents such as propylene glycol, polybutene, and macrogol 1500; antiseptics such as benzoic acid, sodium benzoate, chlorhexidine hydrochloride, sorbic acid, methyl paraoxybenzoate, and butyl paraoxybenzoate; coloring agents such as yellow iron oxide, yellow iron (III) oxide, iron (III) oxide, black iron oxide, carbon black, carmine, β-carotene, copper chlorophyll, Food Blue No.1, Food Yellow No.4, Food Red No. 2, and licorice extract; cooling agents such as fennel oil, d-camphor, dl-camphor, mint oil, d-borneol, and 1-menthol; and perfumes such as spearmint oil, clove oil, vanillin, bergamot oil, and lavender oil.

The pressure-sensitive adhesive composition of the present invention contains the acrylic copolymer obtained by copolymerizing the monomer components containing the above-mentioned monomers (a) and (b) at a content of preferably about 50 weight% or more, more preferably about 70 weight% or more, still more preferably about 90 weight% or more. As long as the content of the acrylic copolymer in the pressure-sensitive adhesive composition falls within such range, a pressure-sensitive adhesive layer having additionally good pressure-sensitive adhesive performance can be formed.

The medical pressure-sensitive adhesive composition of the present invention can contain a drug as desired. The drug described here, which is not particularly limited, is preferably a drug that can be administered to a mammal such as a human being through its skin, i.e., a transdermally absorbable drug. Examples of such drug include drugs that can be transdermally administered of such kinds as a corticosteroid drug, a non-steroidal anti-inflammatory drug, an antirheumatic drug, a sleeping pill, an antipsychotic drug, an antidepressant, a mood stabilizer, a psyohostimulant, an antianxiety drug, an antiepileptic drug, a migraine therapeutic drug, a Parkinson's disease therapeutic drug, a cerebral circulation/metabolism improver, an anti-dementia drug, an autonomic drug, a muscle relaxant, a hypotensive drug, a diuretic drug, a hypoglycemic drug, a hyperlipidemia therapeutic drug, an arthrifuge, a general anesthetic, a local anesthetic, an antibacterial drug, an antifungal drug, an antiviral drug, an anti-parasite drug, a vitamin drug, an angina pectoris therapeutic drug, a vasodilator, an antiarrhythmic drug, an antihistaminic drug, a mediator release inhibitor, a leukotriene antagonist, a female hormone drug, a thyroid hormone drug, an antithyroid drug, an antiemetic, an anti-dizziness drug, a bronchodilator, an antitussive drug, an expectorant, and a smoking cessation adjunct, which can be transdermally administered. Of those, a drug whose stability significantly reduces in a pressure-sensitive adhesive layer containing a carboxyl group can be suitably incorporated in view of the characteristics of the medical pressure-sensitive adhesive composition of the present invention.

It is advantageous to use a basic drug as the above-mentioned drug from such a viewpoint that a transdermally absorbable preparation having high skin permeability is obtained. The basic drug means a drug having a basic group in its molecule. In the case of the medical pressure-sensitive adhesive composition of the present invention containing the acrylic copolymer substantially free of a carboxyl group, for example, the inhibition of the movement of the basic drug in a pressure-sensitive adhesive layer caused by a reaction between the basic group of the basic drug and a carboxyl group can be suppressed. From such viewpoint, the basic drug is preferably a basic drug having a basic nitrogen atom, more preferably a drug having a primary, secondary, or tertiary amino group.

The content of the above-mentioned drug in the medical pressure-sensitive adhesive composition of the present invention can be appropriately set depending on, for example, the kind of the drug and a purpose of its administration, and the age, sex, and symptom of a patient. The content of the above-mentioned drug is preferably about 0.1 weight% to about 40 weight%, more preferably about 0.5 weight% to about 30 weight%. In general, when the content is less than 0.1 weight%, the discharge of an amount of the drug effective for a treatment cannot be expected, and when the content exceeds 40 weight%, a therapeutic effect reaches its limit and an economic disadvantage arises, though a preferred content cannot be uniquely defined because the content varies depending on the selected drug.

The medical pressure-sensitive adhesive composition of the present invention is suitable for the preparation of a medical patch or patch preparation (which may hereinafter be referred to as "patch or the like"). The patch or the like can be a medical pressure-sensitive adhesive sheet prepared by forming, on at least one surface of a sheet-shaped base material (support), a pressure-sensitive adhesive layer with the medical pressure-sensitive adhesive composition of the present invention. Such patch or the like may be a pressure-sensitive adhesive sheet with a base material of such a form that the pressure-sensitive adhesive layer is fixedly provided on one of, or each of both, the surfaces of the support (without any intention of separating the pressure-sensitive adhesive layer from the base material), or may be aso-called base material-less pressure-sensitive adhesive sheet of such a form that the pressure-sensitive adhesive layer is provided on a support having releasability such as a release liner (e.g., releasing paper or a resin sheet whose surface is subjected to a release treatment). The concept of the above-mentioned patch can comprehend those called, for example, an emergency plaster, a rolled plaster, a wound-covering dressing, a pressure-sensitive adhesive tape for fixing a medical device, a pressure-sensltive adhesive bandage, and a surgical tape. In addition, the concept of the patch preparation can comprehend transdermally absorbable preparations such as a cataplasm, a tape drug for transdermal absorption, a matrix-type patch preparation, and a reservoir-type patch preparation. It should be noted that the above-mentioned pressure-sensitive adhesive layer is not limited to a continuously formed layer and may be a pressure-sensitive adhesive layer formed so as to be of a regular or random pattern such as a dot shape or a stripe shape.

The support is not particularly limited. It is preferred that the support be substantially impermeable to a drug or the like. That is, the support is preferably such that a reduction in content of an active component such as the drug, an additive, or the like in the pressure-sensitive adhesive layer does not occur owing to the loss of the active component, additive, or the like from the back surface of the support as a result of its permeation through the support.

Examples of the support include: polyester resins such as polyethylene terephthalate; polyamide-based resins such as nylon; olefin-based resins such as Saran (registered trade mark), polyethylene, polypropylene, and Surlyn (registered trade mark); vinyl-based resins such as an ethylene-vinyl acetate copolymer, polyvinyl chloride, and polyvinylidene chloride; acrylic resins such as an ethylene-ethyl acrylate copolymer; fluorinated carbon resins such as polytertrafluoroethylene; single films of metallic foil and the like, and laminated films thereof . It should be noted that the support has a thickness of typically 10 µm to 500 µm, preferably 10 µm to 200 µm.

The above-mentioned support is preferably a laminated film of a nonporous plastic film formed of any one of the above-mentioned materials and a porous film. Such constitution can improve adhesion (anchoring property) between the support and the pressure-sensitive adhesive layer. In this case, the pressure-sensitive adhesive layer is preferably formed on the side of the porous film. A film that can improve anchoring property with the pressure-sensitive adhesive layer is adopted as the above-mentioned porous film. Specific examples of the film include paper, a woven fabric, a nonwoven fabric, a knitted fabric, and a mechanically perforated sheet. Of those, paper, the woven fabric, or the nonwoven fabric is particularly preferred from the viewpoint of, for example, handleability. The porous film preferably has a thickness of 10 µm to 200 µm. With such thickness, the anchoring property is improved, and the flexibility and attachment operability of the entire patch are excellent. In the case of a thin patch preparation of, for example, a plaster type or a pressure-sensitive adhesive tape type, the porous film preferably has a thickness of 10 µm to 100 µm. In addition, when the woven fabric or the nonwoven fabric is used as the porous film, its mass per unit area is preferably 5 g/m² to 30 g/m² and more preferably 8 g/m² to 20 g/m². It should be noted that, when the support is the above-mentioned laminated film, the nonporous plastic film preferably has a thickness of 1 µm to 25 µm.

Of the above-mentioned supports, a particularly suitable support is a laminated film of a polyester film (preferably a polyethylene terephthalate film) having a thickness of 1.5 µm to 6 µm and a nonwoven fabric made of a polyester (preferably a polyethylene terephthalate) having a mass per unit area of 8 g/m² to 20 g/m²_{.}

When the patch or the like is prepared, a release liner is preferably laminated on the pressure-sensitive adhesive surface of the pressure-sensitive adhesive layer for protecting the pressure-sensitive adhesive surface until the time of use. The release liner is not particularly limited as long as the liner can secure sufficiently light releasability. Specific examples of the release liner include a filmmade of a polyester, a polyvinyl chloride, a polyvinylidene chloride, a polyethylene terephthalate, or the like, paper such as woodfree paper or glassine paper, and a laminated film of woodfree paper, glassine paper, or the like and a polyolefin, the examples each having a surface, which contacts the pressure-sensitive adhesive layer, subjected to a release treatment by applying a silicone resin, a fluorine resin, or the like. The release liner has a thickness of typically 10 µm to 200 µm, preferably 25 µm to 100 µm.

The above-mentioned release liner is preferably a release liner made of a polyester (especially polyethylene terephthalate) resin in terms of barrier property and a price. Further, in this case, its thickness is preferably about 25 µm to 100 µm in terms of handleability.

The above-mentioned pressure-sensitive adhesive layer can be formed by: providing (typically applying) an application liquid for forming the pressure-sensitive adhesive layer to the support or the release liner; and drying the applied liquid to remove a solvent. Alternatively, the pressure-sensitive adhesive layer can be formed by irradiating the pressure-sensitive adhesive composition with an active energy ray (such as UV light) to cure the composition. Such method of forming the pressure-sensitive adhesive layer can be preferably applied to an active energy ray-curable pressure-sensitive adhesive composition prepared by: partially polymerizing the monomer components by any appropriate polymerization method (such as a photopolymerization method) in advance; and compounding the partial polymer with a photopolymerization initiator and a cross-linking agent to be used as required (such as a polyfunctional (meth) acrylate). Such active energy ray-curable pressure-sensitive adhesive composition can be of such composition as to be substantially free of a liquid medium. When the composition contains a liquid medium, the active energy ray is preferably applied after the composition provided for the support has been dried. It should be noted that the application liquid for forming a pressure-sensitive adhesive layer contains the medical pressure-sensitive adhesive composition of the present invention and a proper solvent capable of dissolving the composition. The application liquid may be substantially free of the solvent when the medical pressure-sensitive adhesive composition of the present invention is prescribed so as to be active energy ray-curable.

The application of the pressure-sensitive adhesive composition (substantially the above-mentioned application liquid for forming the pressure-sersitive adhesive layer) can be performed with any appropriate coater such as a gravure roll coater, a reverse roll coater, a kiss-roll coater, a dip roll coater, a bar coater, a knife coater, or a spray coater. The pressure-sensitive adhesive composition is preferably dried under heating from the viewpoints of, for example, the acceleration of a cross-linking reaction and an improvement in production efficiency. A drying temperature is, for example, about 40°C to about 150°C, though the drying temperature varies depending on the kind of the support to which the composition is applied. It should be noted that, in the case of the pressure-sensitive adhesive sheet with a base material, the pressure-sensitive adhesive layer maybe formed by directly applying the pressure-sensitive adhesive composition to the base material, or the pressure-sensitive adhesive layer formed on the release liner may be transferred onto the base material.

When the pressure-sensitive adhesive composition of the present invention is used in a patch or the like, the thickness of the pressure-sensitive adhesive layer is not particularly limited. For example, the thickness of the pressure-sensitive adhesive layer is preferably about 10 µm or more, more preferably about 20 µm or more, still more preferably about 30 µm or more. With such thickness, good pressure-sensitive adhesive performance (such as a good adhesive strength) can be realized. Meanwhile, the thickness is preferably about 400 µm or less, more preferably about 200 µm or less, still more preferably 100 µm or less.

Hereinafter, the present invention is described in more detail by way of examples. However, the present invention is not limited to those shown in such examples. It should be noted that the terms "part(s)" and "%" in the following description refer to "part(s) by weight" and "weight%," respectively unless otherwise stated.

### [Example 1] Pressure-sensitive adhesive composition

First, 70 parts of 2-ethylhexylacrylate (which may herein after be referred to as "2-EHA") as the monomer (a), 10 parts of N-hydroxyethylacrylamide (which may hereinafter be referred to as "HHAA" ) as the monomer (b), 20 parts of N-vinyl-2-pyrrolidone (which may hereinafter be referred to as "N-VP") as the monomer (c), and 333.3 parts of ethyl acetate as a solvent were loaded into a reaction vessel provided with a cooling tube, a nitrogen gas-introducing tube, a temperature gauge, a dropping funnel, and a stirring machine, and then the contents were stirred at room temperature for 1 hour while nitrogen gas bubbling (100 mL/min) was performed. After that, the contents in the reaction vessel were heated, and 0.2 part of 2,2'-azobisisobutyronitrile (AIBN) as a polymerization initiator was added when the temperature of the contents reached 60°C. The temperature of the contents was controlled to be kept at 60°C, and then polymerization was performed in a stream of a nitrogen gas for 6 hours. Next, the temperature was held at 76°C for 15 hours. A pressure-sensitive adhesive composition as a solution of an acrylic copolymer (2-EHA/HEAA/N-VP=70/13/20) (substantially an application liquid for forming a pressure-sensitive adhesive layer, the same holds true for Examples 2 and 3, and Comparative Examples 1 to 3) was obtained by solution polymerization based on the above-mentioned system.

### [Example 2] Pressure-sensitive adhesive composition

A pressure-sensitive adhesive composition as a solution of an acrylic copolymer (2-EHA/HEAA/N-VP=85/10/5) was prepared in the same manner as in Example 1 except that 85 parts of 2-ethylhexyl acrylate as the monomer (a), 10 parts of N-hydroxyethylacrylamide as the monomer (b), and 5 parts of N-vinyl-2-pyrrolidone as the monomer (c) were used as monomer components.

### [Example 3] Pressure-sensitive adhesive composition

A pressure-sensitive adhesive composition as a solution of an acrylic copolymer (2-EHA/HEAA/N-VP=50/10/40) was prepared in the same manner as in Example 1 except that 50 parts of 2-ethylhexyl acrylate as the monomer (a), 10 parts of N-hydroxyethylacrylamide as the monomer (b), and 40 parts of N-vinyl-2-pyrrolidone as the monomer (c) were used as monomer components.

### [Comparative Example 1] Pressure-sensitive adhesive composition

A pressure-sensitive adhesive composition was prepared in the same manner as in Example 2 except that an acrylic copolymer was prepared by using acrylic acid hydroxyethyl ester (which may hereinafter be referred to as "HEA") instead of N-hydroxyethylacrylamide as the monomer (b) (2-EHA/HEA/N-VP=85/10/5) .

### [Comparative Example 2] Pressure-sensitive adhesive composition

Apressure-sensitive adhesive composition was prepared in the same manner as in Example 1 except that an acrylic copolymer was prepared by using 95 parts of 2-ethylhexyl acrylate as the monomer (a) and 5 parts of acrylic acid (which may hereinafter be referred to as "AA") instead of the monomer (b) as monomer components (2-EHA/AA=95/5).

### [Comparative Example 3] Pressure-sensitive adhesive composition

A pressure-sensitive adhesive composition was prepared in the same manner as in Example 1 except that an acrylic copolymer (2-EHA/HEAA/N-VP=50/30/20) was prepared by using: 50 parts of 2-ethylhexyl acrylate as the monomer (a), 30 parts of N-hydroxyethylacrylamide as the monomer (b), and 20 parts of N-vinyl-2-pyrrolidone as the monomer (c) as monomer components; and 100 parts of isopropyl alcohol as a solvent.

A skin pressure-sensitive adhesive sheet was prepared by using any one of the above-mentioned pressure-sensitive adhesive compositions of Examples 1 to 3 and Comparative Examples 1 to 3 (application liquids for forming pressure-sensitive adhesive layers). The skin pressure-sensitive adhesive sheet was prepared by: applying any one of the above-mentioned pressure-sensitive adhesive compositions (application liquids for forming pressure-sensitive adhesive layers) to the release surface of a release liner made of a polyethylene terephthalate (PET) film having a thickness of 75 µm with an applicator; drying the applied composition at 100°C for 3 minutes to form a pressure-sensitive adhesive layer; and attaching the nonwoven fabric surface of a support to the above-mentioned pressure-sensitive adhesive layer. It should be noted that a laminate of a PET film having a thickness of 2 µm and a PET nonwoven fabric having a mass per unit area of 14 g/m² was used as the support.

### <Retaining strength>

The skin pressure-sensitive adhesive sheets prepared in the foregoing were each evaluated for its retaining strength as an indicator for a cohesive strength as described below. That is, a test piece was produced by cutting each of the above-mentioned skin pressure-sensitive adhesive sheets into a size measuring 10 mm wide by 50 mm long. A clean Bakelite plate washed with a clean waste impregnated with acetone was used as an adherend. The release liner was released from the above-mentioned test piece, and then the remainder was attached to the above-mentioned adherend so as to have an adhesion area measuring 10 mm wide by 20 mm long by rolling a 2-kg roller once in a reciprocating manner. After the resultant had been stored at 40 °C for 20 minutes, the above-mentioned adherend was drooped under an environment having a temperature of 40°C. A load of 300 g was applied to the free end of the above-mentioned test piece, and then a time required for the test piece to fall from the adherend was measured. It should be noted that the skin pressure-sensitive adhesive sheet prepared by using the pressure-sensitive adhesive composition of Example 1 was evaluated by using a load of 500 g because, when the load of 300 g was used, the test piece did not fall for 24 hours or more and hence no difference could be observed. In addition, the skin pressure-sensitive adhesive sheet prepared by using the pressure-sensitive adhesive composition of Comparative Example 2 was subjected to measurement by using each of both the loads of 300 g and 500 g in order that the sheet might be evaluated in an additionally detailed manner.

### <Tube fixability test>

Each of the above-mentioned pressure-sensitive adhesive sheets was cut into a sample measuring 12 mm wide by 55 mm long. In addition, a product obtained by fixing a defatted, stretched collagen film on a flat table with a double-faced tape was used as a skinmodel. Atubemade of vinyl chloride having an inner diameter of 3 mm, an outer diameter of 5 mm, and a length of 70 mm was bent into an arc having a diameter of 20 µm, and was then fixed on the above-mentioned collagen film with the above-mentioned sample so that the sample might intersect two sites of the tube. Adhesion states after lapses of 3 hours and 19 hours from the fixation were j udgedby visual observation. Tube fixability was evaluated as being ○ when the adhesion was observed even after the lapse of 19 hours. On the other hand, the tube fixability was evaluated as being × when the tube was observed to fall before the lapse of 19 hours. In addition, traveling distances between tube terminals after the lapses of 3 hours and 19 hours were measured. Since a distance between the tube terminals immediately after the arcuate fixation was 23 mm, the traveling distance between the tube terminals was determined from the following equation by using the foregoing distance as an initial value.
Traveling distance between tube terminals (mm)=[distance between terminals at each time]-[initial value (23 mm)]

Table 1 shows the results of the above-mentioned retaining strength evaluation and tube fixability test. In addition, Table 1 shows the glass transition temperature (Tg) of each of the above-mentioned pressure-sensitive adhesive compositions of Examples 1 to 3 and Comparative Examples 1 to 3 calculated from the Tg of a homopolymer constituted of each monomer together with the results.

**Table 1**

| Sample | Tg (°C) | Retaining strength (minutes) | | Tube fixability | | |
|---|---|---|---|---|---|---|
| | | Load 300 g | Load 500 g | Adhesion time | Evaluation | Traveling distance between tube terminals after 3 hours (mm) |
| Example 1 | -42.0 | | 160.9 | >19 | ○ | 0.3 |
| Example 2 | -56.1 | 21.6 | - | >19 | ○ | 8.7 |
| Example 3 | -20.2 | - | >1440 | >19 | ○ | |
| Comparative Example 1 | -61.5 | 7.3 | - | 3 to 19 | × | 18.3 |
| Comparative Example 2 | -65.2 | 35.9 | 11.1 | >19 | ○ | 2.0 |
| Comparative Example 3 | -15.5 | * | | >3 | × | - |

| | | | | | | |
|---|---|---|---|---|---|---|
| *The test piece was not attached to the adherend. | | | | | | |

As is apparent from Table 1, the skin pressure-sensitive adhesive sheet prepared by using the pressure-sensitive adhesive composition of Example 1 was observed to have a high adhesive strength and a high cohesive strength because the sheet did not fall for 160 minutes or more even when the load of 500 g was used in the evaluation for a retaining strength. In addition, the above-mentioned skin pressure-sensitive adhesive sheet showed sufficient skin fixability in the tube fixability test. The skin pressure-sensitive adhesive sheet using the pressure-sensitive adhesive composition of Example 2 showed a sufficient adhesive strength and a sufficient cohesive strength when the load of 300 g was used. In addition, the sheet had good tube fixability. The skin pressure-sensitive adhesive sheet using the pressure-sensitive adhesive composition of Comparative Example 1 fell even when the load of 300 g was used. Accordingly, the sheet had an insufficient skin adhesive strength and an insufficient cohesive strength. It should be noted that the skin pressure-sensitive adhesive sheet prepared by using the pressure-sensitive adhesive composition of Comparative Example 2 had a sufficient adhesive strength, a sufficient cohesive strength, and sufficient tube fixability. However, the sheet had low skin permeability in a skin permeability test to be described later, though no data on the skin permeability was shown.

### [Example 4] Pressure-sensitive adhesive composition

Pramipexole (PRA) as a drug was added to the solution of the acrylic copolymer prepared in Example 1 in an amount of 5.3 parts with respect to 100 parts (solid content) of the acrylic copolymer. Thus, a pressure-sensitive adhesive composition was prepared. It should be noted that pramipexole has one primary amino group and one secondary amino group in its molecule as shown in the following formula (III).

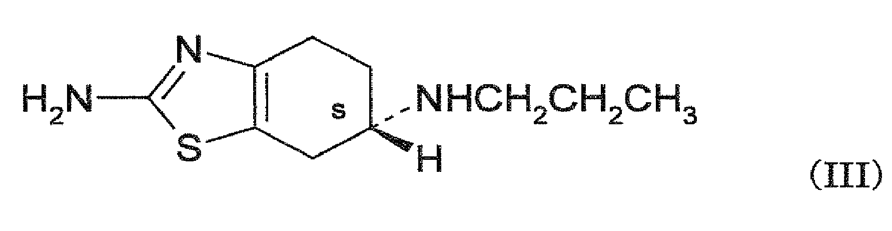

### [Comparative Example 4] Pressure-sensitive adhesive composition

Under an inert gas atmosphere, 72 parts of 2-ethylhexyl acrylate, 25 parts of N-vinyl-2-pyrrolidone, 3 parts of acrylic acid, and 0.2 part of azobisisobutyronitrile were added to ethyl acetate, and then the contents were mixed. The mixture was subj ected to solution polymerization at 60°C. Thus, a solution (solid content: 28 weight%) of an acrylic copolymer (2-EHA/AA/N-VP=72/3/25) was obtained. Pramipexole (PRA) as a drug was added to the above-mentioned solution of the acrylic copolymer in an amount of 5.3 parts with respect to 100 parts (solid content) of the acrylic copolymer. Thus, a pressure-sensitive adhesive composition was prepared.

A skin pressure-sensitive adhesive sheet was prepared by using each of the above-mentioned pressure-sensitive adhesive compositions of Example 4 and Comparative Example 4. That is, one of the above-mentioned pressure-sensitive adhesive compositions was applied to the release surface of a release sheet made of a polyethylene terephthalate (PET) film having a thickness of 75 µm with an applicator, and was then dried at 80°C for 5 minutes. After that, a PET film having a thickness of 25 µm as a support was attached to the resultant. Thus, a skin pressure-sensitive adhesive sheet drug was prepared.

### <Evaluation for drug permeability>

Each of the above-mentioned skin pressure-sensitive adhesive sheets was evaluated for its drug permeability. That is, a skin extirpated from a hairless mouse was mounted on a vertical diffusion cell, each of the above-mentioned preparations was applied to a donor cell, and a physiological saline was applied to a receiver cell. A receiver liquid was recovered at a predetermined time interval, and then the amount of pramipexole that had permeated was determined by high performance liquid chromatography (HPLC). HPLC was performed under the following conditions.

### (HPLC measurement conditions)

| | |
|---|---|
| Column: | TSK-gel ODS-80Ts QA (5 µm, 150×4.6 mm I.D.; TOSOH) |
| Mobile phase: | A 1% aqueous solution of triethylamine (having a pH of 7.0)/methanol (80:20) |
| Column temperature: | 40°C |
| Flow rate: | 0.7 mL/min |
| Detector: | A UV absorptiometer (having a measurement wavelength of 262 nm) |

FIG. **1** illustrates the results of the evaluation for drug permeability. As is apparent from FIG. **1**, pramipexole was observed to permeate the skin sufficiently in the skin pressure-sensitive adhesive sheet prepared by using the medical pressure-sensitive adhesive composition of Example 4. In contrast, nearlyno permeation of pramipexole through the skin was observed in the skin pressure-sensitive adhesive sheet prepared by using the pressure-sensitive adhesive composition of Comparative Example 4.

As described above, according to the present invention, there can be provided the following medical pressure-serfsitive adhesive composition. That is, when the composition contains a medical active component such as a drug, the denaturation or the like of the composition due to a reaction with such component is suppressed, and the composition does not threaten to reduce the effectiveness of the component. In addition, when the pressure-sensitive adhesive layer of a patch or patch preparation is formed, the composition can show good fixability for a skin surface and impart excellent fixability to a medical device such as a catheter. The medical pressure-sensitive adhesive composition of the present invention is particularly suitable for applications such as medical patches and patch preparations.

Many other modifications will be apparent to and be readily practiced by those skilled in the art without departing from the scope and spirit of the invention. It should therefore be understood that the scope of the appended claims is not intended to be limited by the details of the description but should rather be broadly construed.

## Claims

1. A medical pressure-sensitive adhesive composition comprising an acrylic copolymer obtained by copolymerizing monomer components containing (a) at least one kind of a monomer of a (meth) acrylic acid alkyl ester and (b) at least one kind of a monomer of an N-hydroxyalkyl (meth) acrylamide, wherein:
a content of the (meth) acrylic acid alkyl ester monomer (a) with respect to a total amount of the monomer components is 50 weight% to 99. 9 weight% and a content of the N-hydroxyalkyl (meth) acrylamide monomer (b) with respect to the total amount is 0.1 weight% to 20 weight%; and
the monomer components are substantially free of a monomer having a carboxyl group.

2. A medical pressure-sensitive adhesive composition according to claim 1, wherein the (meth) acrylic acid alkyl ester (a) has an alkyl group having 1 to 20 carbon atoms.

3. A medical pressure-sensitive adhesive composition according to claim 1, wherein the N-hydroxyalkyl(meth)acrylamide (b) has a hydroxyalkyl group having 2 to 4 carbon atoms.

4. A medical pressure-sensitive adhesive composition according to claim 1, wherein a total content of the (meth)acrylic acid alkyl ester monomer (a) and the N-hydroxyalkyl(meth)acrylamide monomer (b) with respect to the total amount of the monomer components is 60 weight% or more.

5. A medical pressure-sensitive adhesive composition according to claim 1, wherein the (meth)acrylic acid alkyl ester (a) comprises at least one selected from the group consisting of butyl acrylate, 2-ethylhexyl acrylate, butyl methacrylate, and 2-ethylhexyl methacrylate.

6. A medical pressure-sensitive adhesive composition according to claim 1, wherein the N-hydroxyalkyl(meth)acrylamide (b) comprises at least one selected from the group consisting of N-(2-hydroxyethyl)acrylamide and N-(2-hydroxyethyl)methacrylamide.

7. A medical pressure-sensitive adhesive composition according to claim 1, wherein the acrylic copolymer has a glass transition temperature (Tg) of -10°C or less.

8. Use of the medical pressure-sensitive adhesive composition according to anyone of Claims 1 to 7 in a medical patch or patch preparation.
